(19) Europäisches Patentamt
European Patent Office
Office européen des brevets

(11) Publication number: 0 005 914 A1

# (12) EUROPEAN PATENT APPLICATION

(21) Application number: 79300779.0

(22) Date of filing: 08.05.79

(51) Int. Cl.$^2$: C 07 D 498/04 //C07D401/12, (C07D498/04, 263/00, 205/00)

(30) Priority: 30.05.78 GB 2404678

(43) Date of publication of application: 12.12.79 Bulletin 79/25

(84) Designated Contracting States: BE CH DE FR GB IT NL SE

(71) Applicant: BEECHAM GROUP LIMITED
Beecham House Great West Road
Brentford, Middlesex(GB)

(72) Inventor: Brooks, Gerald
6 Worcester Road
Reigate Surrey(GB)

(72) Inventor: Davies, John Sydney
17 Beaufort Road
Reigate Surrey(GB)

(74) Representative: Cole, William Gwyn et al,
Beecham Pharmaceuticals Yew Tree Bottom Road
Epsom Surrey KT18 5XQ(GB)

## (54) Chemical process for the preparation of beta-lactamase inhibitors, and so obtained compounds.

(57) This invention provides a process for the preparation of thioether derivatives of clavulanic acid, comprising the reaction of an ester of clavulanic acid, a sulphenimide (II):

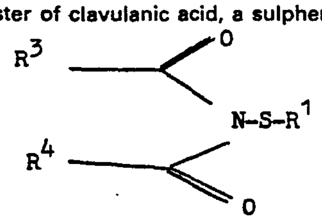

(II)

and a phosphine $PR^5R^6R^7$.

EP 0 005 914 A1

TITLE MODIFIED
see front page

## Chemical Process For the Preparation of β-Lactamase Inhibitors

British Patent Application 03892/76-19002/76 (see also Belgian Patent No. 850779) discloses thioether derivatives of clavulanic acid, pharmaceutical compositions containing them and a process for their preparation. The disclosed process comprised the reaction of an ester of clavulanic acid with a thiol and thereafter if desired converting the thus formed ester of the thioether into the corresponding free acid or its salt. Although the process disclosed is very versatile leading to the preparation of a wide class of thioethers it suffers from the disadvantage of giving only relatively poor yields. The later French Published Patent Application No. 2,342,292 also discloses the preparation of thioethers of clavulanic acid by the reaction of a sulphur nucleophile with an ester of 9-halo or 9-acyloxydeoxyclavulanic acid.

It is desirable to find a synthesis that has acceptable yields and is convenient to operate. A synthesis has now been found that has the advantage that it may be carried out at ambient temperature if desired, does not involve the use of foul smelling thiols at the coupling stage but instead employs easily weighable, solid sulfenimide intermediates. The intermediates used in this process are also conveniently accessible

and used, for example by the methods of M.Behforouz and J.E. Kenwood, J. Org. Chem., 34, 51 (1969) or K.A.M. Walker, Tet. Lett., 4475 (1977). The use of this process for preparing aryl thioethers offers the further advantage of particularly good yields.

The present invention provides a process for the preparation of thioether derivatives of deoxyclavulanic acid of the formula (I):

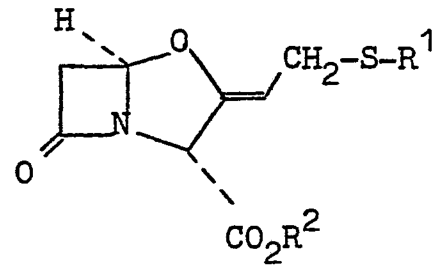

(I)

wherein $R^1$ is the residue of a thiol $R^1SH$ and $R^2$ is a group such that $CO_2R^2$ represents a carboxylic acid moiety or a salt or ester thereof; which process comprises the reaction of a compound of the formula (II):

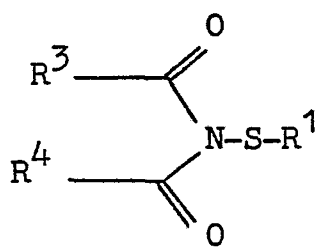

(II)

with a compound of the formula (III):

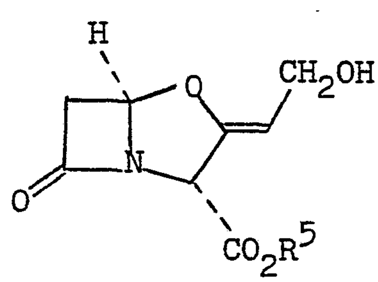

(III)

and a compound of the formula (IV):

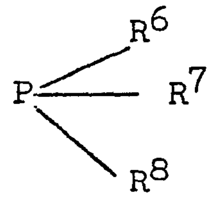

(IV)

in which compounds $R^1$ is as defined in relation to formula (I); $R^3$ and $R^4$ are moieties such that the compound of the formula (II) is a sulfenimide; $R^5$ is a group such that the compound of the formula (III) is an ester of clavulanic acid; and $R^6$, $R^7$ and $R^8$ are groups such that the compound of the formula (IV) is a phosphine; and thereafter if desired converting the thus formed compound of the formula (I) wherein $R^2$ is a group such that $CO_2R^2$ represents an esterified carboxylic acid moiety to a corresponding compound wherein $R^2$ is a moiety such that $CO_2R^2$ represents a carboxylic acid moiety or a salt thereof.

Suitable groups $R^1$ include hydrocarbon groups and hydrocarbon groups substituted by a group of the sub-formulae $OR^9$, $O.CO.R^9$, $CO.R^9$, $CO_2R^9$, $NH.CO.R^9$, $NR^{10}.COR^9$ and $NR^{10}CO_2R^9$ wherein $R^9$ is a lower alkyl group or lower aralkyl group and $R^{10}$ is a lower alkyl group.

When used herein the term "hydrocarbon" includes alkyl, alkenyl, phenyl and phenylalkyl groups. When used herein the term "lower" means that the group contains up to 4 carbon atoms. When used herein the term "aralkyl" means a lower alkyl group substituted by a phenyl group.

Certain favourable groups $R^1$ include the methyl, ethyl, n-propyl, n-butyl, phenyl, benzyl, allyl, 2-methoxyethyl, 4-methoxyphenyl, 4-methoxybenzyl and the like.

Preferred groups $R^1$ include optionally substituted phenyl groups.

Further preferred groups $R^1$ are optionally substituted aromatic heterocyclic groups, such as pyridyl, furyl, thienyl and the like.

Thus it will be realised that in a particularly preferred aspect of this invention, $R^1$ is an optionally substituted aromatic group.

Suitable groups $-CO_2R^2$ include those of the sub-formulae $-CO_2A^1$ or $-CO_2CHA^2A^3$ wherein $A^1$ is a lower alkyl group optionally substituted by a group $OA^4$, $O.CO.A^4$ or $O.CO.O.A^4$ wherein $A^4$ is a lower alkyl or phenyl group; $A^2$ is a lower alkenyl, lower alkynyl, phenyl group or phenyl group substituted by a halogen atom or nitro, $A^5$, $OA^5$ or $O.CO.A^5$ group where $A^5$ is a lower alkyl group; and $A^3$ is a hydrogen atom or a phenyl group or a phenyl group substituted by a halogen atom or a nitro, $A^5$, $OA^5$ or $O.CO.A^5$ group where $A^5$ is a lower alkyl group; or $-CHA^2A^3$ represents a phthalidyl group.

Suitably the $-CO_2R^2$ group is one readily cleaved by mild base, for example a lower alkyl or lower alkoxymethyl group.

Suitably the $-CO_2R^2$ group is one readily cleaved by hydrogenolysis, for example a benzyl group optionally substituted by a halogen atom or a nitro, $A^5$, $OA^5$ or $O.CO.A^5$ group where $A^5$ is a lower alkyl group. The benzyl, p-methoxybenzyl and p-nitrobenzyl group are particularly favoured.

The groups $R^3$ and $R^4$ will normally be selected from hydrocarbon groups which may be linked together. Particularly apt groups $R^3$ and $R^4$ include lower alkyl groups optionally linked to form part of a 5- or 6-membered ring.

Preferred compounds of the formula (II) include those of the formulae (V) and (VI):

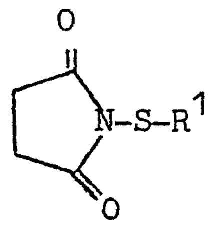

(V)

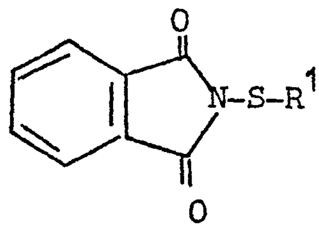

(VI)

wherein $R^1$ is as defined in relation to formula (I).

The compounds of the formula (V) are particularly suitable as a by-product of the reaction is water-soluble and may thus be readily removed by washing with water.

Suitable values for $R^6$, $R^7$ and $R^8$ include hydrocarbon groups of up to 7 carbon atoms and hydrocarbon groups substituted by a group of the sub-formulae $OR^{11}$ or $O.CO.R^{11}$ where $R^{11}$ is a lower alkyl group.

Most suitably $R^6$, $R^7$ and $R^8$ each have the same meaning.

Favoured values for $R^6$, $R^7$ and $R^8$ include methyl, ethyl, n-propyl, n-butyl, phenyl, 4-methoxyphenyl and benzyl.

A particularly suitable compound of the formula (IV) is tri-n-butyl-phosphine.

The reaction of the compounds of the formulae (II), (III) and (IV), may take place at any convenient non-extreme temperature, for example -10°C to 50°C, more usually 0° to 40°C, generally 10° to 30°C and very conveniently at ambient (about 15-25°C). The reaction is generally carried out in an aprotic medium. Suitable solvents include hydrocarbon solvents such as benzene,

toluene and the like.

If desired the reaction may be carried out under an inert gas such as nitrogen.

Once the reaction is complete, for example as indicated by tlc, the mixture may be washed with water to remove the water-soluble products, and the organic layer dried and evaporated. The initial product may be further purified by chromatography if desired, for example over silica eluting with ethyl acetate/petroleum ether or the like.

Once formed the ester of the thioether may be converted to the corresponding acid or salt in conventional manner, for example as described in Belgian Patent No. 847045.

Example 1

p-Nitrobenzyl 9-phenylthiodeoxyclavulanate

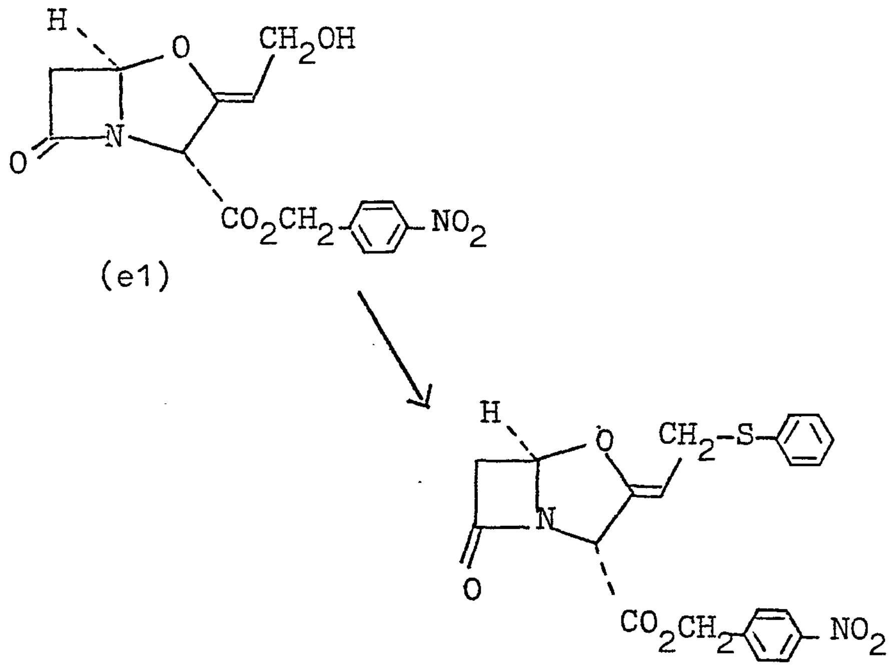

(e1)

(e2)

A stirred solution of tri-n-butylphosphine (0.088 g, 0.435 mmole) in dry benzene (1 ml) under nitrogen was treated with a solution of N-(phenylthio)-succinimide (0.090 g, 0.435 mmole) in dry benzene (3 ml). After 5 minutes solid p-nitrobenzyl clavulanate (e1) (0.144 g, 0.43 mmole) was added and the mixture stirred for 30 minutes, washed with water (2 x 5 ml), dried ($MgSO_4$) and evaporated. Chromatography of the residue on silica, eluting with ethyl acetate/petroleum ether 1/4 yielded p-nitrobenzyl 9-phenylthiodeoxyclavulanate (e2) (0.118 g, 64%) as an oil. $[\alpha]_D^{20}$ + 9.0° (c. 1.0; $CHCl_3$). $\nu_{max}$ ($CHCl_3$) 1805, 1755, 1690, 1525 and 1350 $cm^{-1}$; $\lambda_{max}$ (dioxan) 258 nm (14600); $\delta$ ($CDCl_3$): 2.87 (1H, d, J 17Hz, 6β-CH), 3.42 (1H, dd, J 17 and 2.5Hz, 6α-CH), 3.58 (2H, d, J 8Hz, 9-$CH_2$), 4.75 (1H, dt, J 8 and 1 Hz 8-CH),

5.04 (1H, d, J 1Hz, 3-CH), 5.18 (2H, s, $CH_2$ Ar), 5.57 (1H, d, J 2.5Hz, 5-CH), 7.1-7.5 (5H, m, -SPh), 7.42 and 8.17 (4H, 2d, p-$NO_2$Ph-).

Example 2

p-Nitrobenzyl 9-phenylthiodeoxyclavulanate

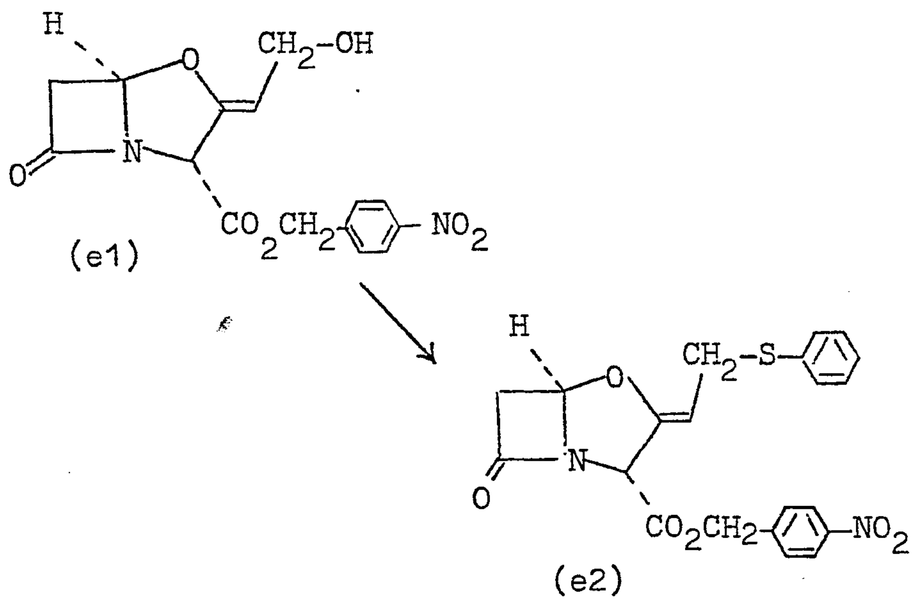

(e1)

(e2)

A stirred solution of tri-n-butylphosphine (0.67 g, 3.3 mmole) in dry benzene (10 ml) under nitrogen was treated with solid N-(phenylthio)-phthalimide (0.842 g, 3.3 mmole). After 5 minutes solid p-nitrobenzyl clavulanate (e1) (1.0 g, 3 mmole) was added and stirring continued for 1 hour. The solution was washed with water (2 x 10 ml), dried ($MgSO_4$) and evaporated and the residue chromatographed on silica, eluting with ethyl acetate/petroleum ether 1/4. The product thus obtained was taken up in tetrachloromethane and crystalline phthalimide filtered off. The filtrate contained the required p-nitrobenzyl 9-phenylthiodeoxyclavulanate (e2) which was obtained by evaporation as an oil (0.74 g, 50%) identical to the product of Example 1.

Example 3

Sodium 9-phenylthiodeoxyclavulanate

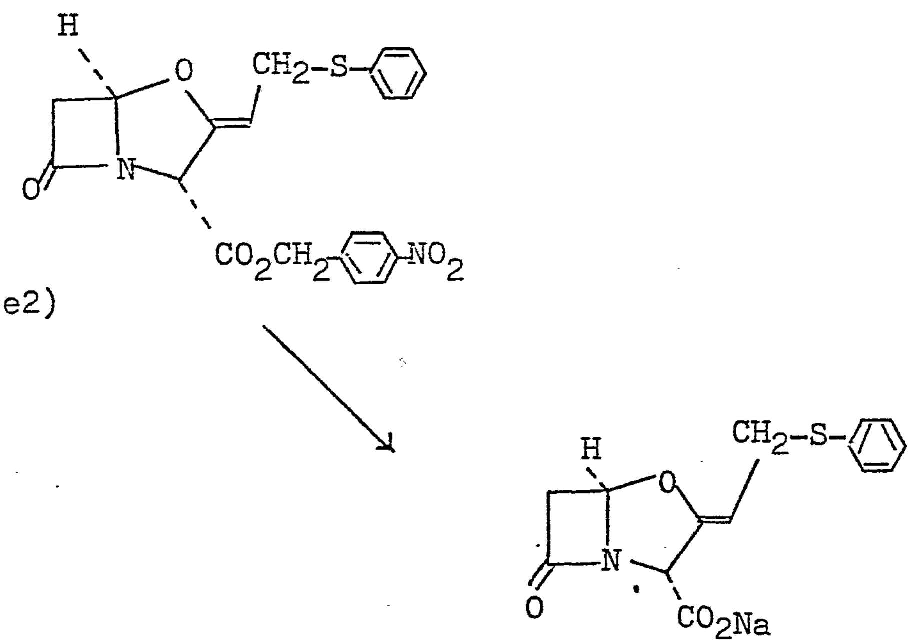

(e2)

(e3)

A solution of p-nitrobenzyl 9-phenylthiodeoxyclavulanate (e2) (1.05 g, 2.46 mmole) in tetrahydrofuran (10 ml) and a solution of sodium bicarbonate (0.206 g, 2.46 mmole) in water (8 ml) were added to a prehydrogenated suspension of 10% Pd/C (1.3 g) in tetrahydrofuran (20 ml) and the mixture hydrogenated at 1 atmosphere for 2 hours. After filtration, the solution was washed with ether (3 x 10 ml), adjusted to pH 7 (dilute hydrochloric acid) and freeze dried to yield the required sodium 9-phenylthiodeoxyclavulanate (e3) (0.34 g, 44%) as a solid. $\nu_{max}$ (nujol) 1788, 1692 and 1615 $cm^{-1}$; $\delta$ ($D_2O$; HOD at 4.60) 2.57 (1H, d, J 17Hz, 6β-C<u>H</u>), 3.2-3.9 (3H, m, 6α-C<u>H</u> and 9-C$\underline{H}_2$), 4.76 (1H, s, 3-C<u>H</u>), 5.43 (1H, d, J 2.5Hz, 5-C<u>H</u>), 7.1-7.5 (5H, m, Ph-H). 8-C<u>H</u> obscured by HOD.

Example 4

Lithium 9-phenylthiodeoxyclavulanate

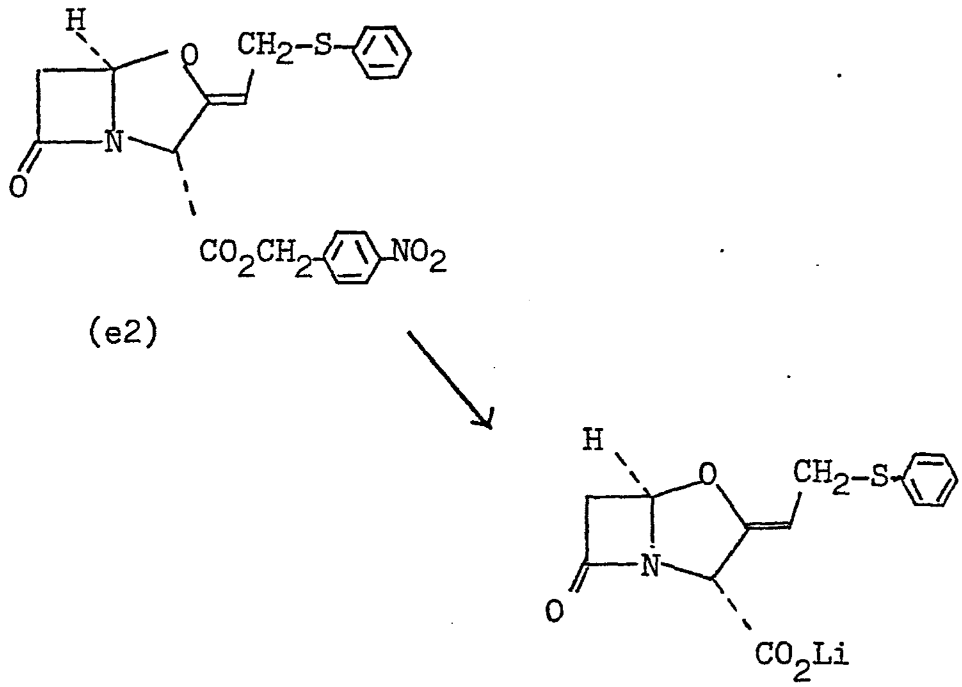

(e2)

(e4)

A solution of p-nitrobenzyl 9-phenylthiodeoxyclavulanate (e2) (1.45 g, 3.50 mmole) in tetrahydrofuran (100 ml) was added to a prehydrogenated suspension of 10% Pd/C (1.7 g) in tetrahydrofuran (100 ml) and the mixture hydrogenated at 1 atmoshpere for 1½ hours. After filtration, the solution was evaporated to 20 ml, ice cooled and treated with a solution of lithium carbonate (0.122 g, 1.65 mmole) in water (20 ml). The resulting cloudy solution was washed with ether (3 x 40 ml) filtered through celite, adjusted to pH 7 (dilute hydrochloric acid) and freeze dried to yield the required lithium 9-phenylthiodeoxyclavulanate (e4) (0.72 g, 71%) as a solid. $[\alpha]_D^{20}$ + 46.7° (c. 0.94; $H_2O$); $\nu_{max}$ (KBr) 1770, 1690 and 1615 $cm^{-1}$; δ ($D_2O$/hexamethyldisilane) 2.56 (1H, d, J 18 Hz, 6β-CH), 3.1-3.8 (3H, m, 6α-CH and 9-$CH_2$), 4.76 (1H, s, 3-CH), 5.43 (1H, d, J 3Hz, 5-CH), 7.0-7.5 (5H, m, Ph-H). 8-CH obscured by HOD.

Example 5

p-Nitrobenzyl 9-(pyrid-4'-ylthio)-9-deoxyclavulanate

(a) N-(pyrid-4-ylthio)- succinimide

Dipyrid-4-yl disulfide ("Aldrithiol-4", 2.2g, 0.01 mole) and N-bromo-succinimide (1.78g, 0.01 mole) were refluxed in dry benzene (20 ml) for 45 minutes and the solution poured into 200 ml petrol. The solid was filtered off and crystallised from ethanol to provide the title compound (0.25g).

$\nu_{max}$ ($CHCl_3$) 1740 $cm^{-1}$; $\delta$($CDCl_3$/trace DMSO) 3.00 (4H, s, $-CH_2CH_2-$), 7.0-7.2 and 8.4-8.7 (4H,2m, pyridyl-H).

(b) p-Nitrobenzyl 9-(pyrid-4'-ylthio)-9-deoxyclavulanate

N-(pyrid-4-ylthio) succinimide (0.100g,0.48 mmole) was added to a solution of tri-n-butylphosphine (0.118ml, 0.48 mmole) in THF (5ml) under nitrogen and stirred 5 minutes. p-Nitrobenzyl clavulanate (0.100g,0.30 mmole) was added and stirring continued for 30 minutes. The solution was diluted with ethyl acetate (30 ml), washed with water (2 x 20 ml), dried over $MgSO_4$ and evaporated. The residue was chromatographed on silica (12g), eluting with ethyl acetate/petrol 2:1, to yield the title compound as an oil (0.07g,55%). $[\alpha]^{20}_{D}$ + 7.7° (c. 1.3; $CHCl_3$); $\nu$max ($CHCl_3$) 1807, 1758 and 1694 $cm^{-1}$; $\lambda$max (dioxan) 260.5 nm (19400); $\delta$($CDCl_3$) 3.07 (1H, d, J 17Hz, 6β-C<u>H</u>), 3.4 - 3.9 (3H, m, 6α-C<u>H</u> and 9-C$\underline{H}_2$), 4.74 (1H, dt, J 8 and 1Hz, 8-C<u>H</u>), 5.11 (1H, broad s, 3-C<u>H</u>), 5.19 (2H, s, -C$\underline{H}_2$Ar), 5.74 (1H, d, J 2.5Hz, 5-C<u>H</u>), 7.0-7.1 and 8.3-8.4 (4H, 2m, pyridyl-H), 7.41 and 8.15 (4H, 2d, p-nitrophenyl-H).

Example 6

Lithium 9-(pyrid-4'-ylthio)-9-deoxyclavulanate

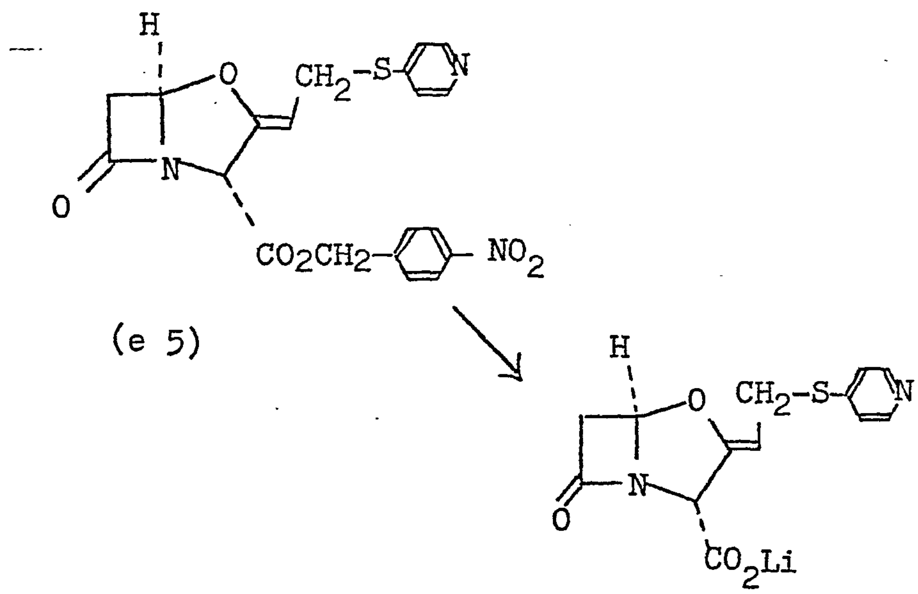

(e 5)

(e 6)

A solution of p-nitrobenzyl 9-(pyrid-4'-ylthio)-9-deoxyclavulanate (0.25g 0.585 mmole) in THF (80ml) was added to a pre-hydrogenated suspension of 10% Pd/C (0.38g) in THF (20ml). The mixture was hydrogenated at 1 atmosphere for 1 hour, filtered through celite and the filtrate evaporated to 20ml. A solution of lithium carbonate (0.021g, 0.284 mmole) in water (40ml) was added dropwise with stirring and the resulting solution washed with ether (3 x 50ml), adjusted to pH7 with 1N hydrochloric acid, filtered through celite and freeze-dried to provide the title compound as a solid (0.10g, 57%). $[\alpha]_D^{20}$ + 24.2° (c 1.0; DMSO); $\nu_{max}$ (KBr) 1785, 1693 and 1615cm$^{-1}$; $\lambda$max ($H_2O$) 215nm (s) (12800) and 266.5nm (8480); $\delta$ (DMSO) 2.89 (1H, d, *J* 16.5Hz, 6β-C<u>H</u>) 3.3-4.0 (3H, m, 6α-C<u>H</u> and 9-C<u>H</u>$_2$), 4.46 (1H, s, 3-C<u>H</u>), 4.59 (1H, t, *J* 8Hz, 8-C<u>H</u>), 5.64 (1H, d, *J* 2.5Hz, 5-C<u>H</u>), 7.20 and 8.31 (4H, 2d, pyridyl-H).

Claims

1. A process for the preparation of thioether derivatives of deoxyclavulanic acid of the formula (I):

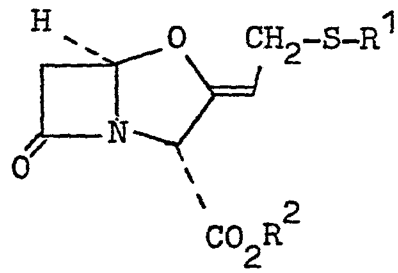

(I)

wherein $R^1$ is the residue of a thiol $R^1SH$ and $R^2$ is a group such that $CO_2R^2$ represents a carboxylic acid moiety or a salt or ester thereof; which process comprises the reaction of a compound of the formula (II):

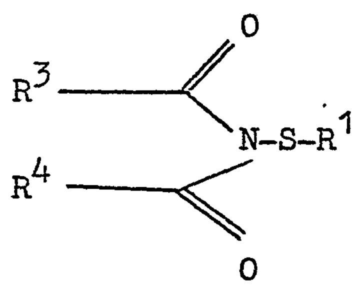

(II)

with a compound of the formula (III):

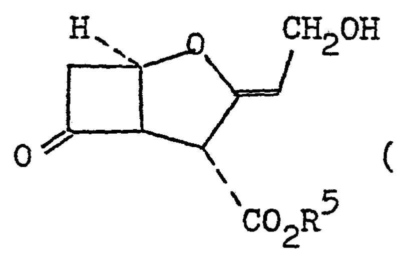

(III)

and a compound of the formula (IV):

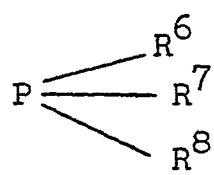

(IV)

in which compounds $R^1$ is a defined in relation to formula (I); $R^3$ and $R^4$ are moieties such that the compound of the formula (II) is a sulfenimide; $R^5$ is a group such that the compound of the formula (III) is an ester of clavulanic acid; and $R^6$, $R^7$ and $R^8$ are groups such that the compound of the formula (IV) is a phosphine; and thereafter if desired converting the thus formed compound of the formula (I) wherein $R^2$ is a group such that $CO_2R^2$ represents an esterified carboxylic acid moiety to a corresponding compound wherein $R^2$ is a moiety such that $CO_2R^2$ represents a carboxylic acid moiety or a salt thereof.

2. A process according to claim 1 wherein $R^1$ is a hydrocarbon group or a hydrocarbon group or a hydrocarbon group substituted by a group of the sub-formula $OR^9$, $O.CO.R^9$, $CO.R^9$, $CO_2R^9$, $NR^{10}.COR^9$ and $NR^{10}CO_2R^9$ wherein $R^9$ is a lower alkyl group or lower aralkyl group and $R^{10}$ is a lower alkyl group.

3. A process according to claim 1 wherein $R^1$ is a methyl, ethyl, n-propyl, n-butyl, phenyl, benzyl, allyl, 2-methoxyethyl, 4-methoxyphenyl or 4-methoxybenzyl.

4. A process according to claim 1 wherein $R^1$ is an optionally substituted phenyl group.

5. A process according to claim 1 wherein $R^1$ is an optionally substituted aromatic heterocyclic group.

6. A process according to any of claims 1 - 5 wherein the compound of the formula (II) is a compound of the formula (V) or (VI):

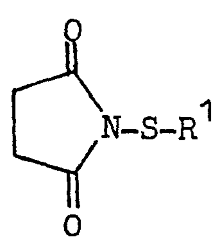

(V)

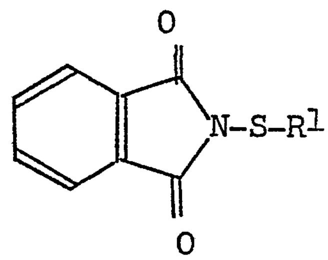

(VI)

7. A process according to any of claims 1 - 6 wherein the compound of the formula (IV) is tri-n-butylphosphine.

8. A process according to any of claims 1 - 7 substantially as described in any example herein.

9. A compound of the formula (I) as defined in claim 1 when prepared by the process of any of claims 1 - 8.

0005914

European Patent Office

# EUROPEAN SEARCH REPORT

Application number

EP 79 30 0779

| DOCUMENTS CONSIDERED TO BE RELEVANT | | | CLASSIFICATION OF THE APPLICATION (Int. Cl.³) |
|---|---|---|---|
| Category | Citation of document with indication, where appropriate, of relevant passages | Relevant to claim | |
| D | TETRAHEDRON LETTERS, No. 51, December 1977 Pergamon Press OXFORD (GB) K.A.M. WALKER: "A convenient preparation of thioethers from alcohols" pages 4475-4478<br>* complete document * | 1,6,7 | C 07 D 498/04 // C 07 D 401/12 (C 07 D 498/04 263/00 205/00) |
| | --- | | |
| | SYNTHESIS, INTERNATIONAL JOURNAL OF METHODS IN SYNTHETIC ORGANIC CHEMISTRY, NO.4, April 1974 Georg Thieme Verlag STUTTGART (DE) M. FURUKAWA: "Preparation of Unsymmetrical Sulfides" pages 282-283<br>* complete article * | 1,6 | TECHNICAL FIELDS SEARCHED (Int.Cl.³)<br>C 07 D 498/04 |
| | --- | | |
| D,A | FR - A - 2 342 292 (GLAXO)<br>* pages 80-89; claims 1,8,19,32 * | 1,9 | |
| | --------- | | |

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 20-08-1979 | CHOULY |

EPO Form 1503.1 06.78